# EUROPÄISCHE PATENTSCHRIFT

(11) **EP 2 247 251 B1**
(45) Veröffentlichungstag und Bekanntmachung des Hinweises auf die Patenterteilung: **04.05.2016**
(21) Anmeldenummer: 09714870.4
(22) Anmeldetag: 20.02.2009
(51) Int. Cl.: A61C 1/00, A61B 18/20, A61B 18/00, A61B 17/00, A61B 18/26

(54) **LASERBEARBEITUNGSGERÄT ZUR BEARBEITUNG VON BIOLOGISCHEM GEWEBE**
LASER PROCESSING DEVICE FOR PROCESSING BIOLOGICAL TISSUE
APPAREIL DE TRAITEMENT AU LASER POUR LE TRAITEMENT DE TISSU BIOLOGIQUE

(30) Priorität: 29.02.2008 DE 102008011811
(43) Veröffentlichungstag der Anmeldung: 10.11.2010
(73) Patentinhaber: Lumera Laser GmbH, 67661 Kaiserslautern (DE); Kasenbacher, Anton, 83278 Traunstein (DE)
(72) Erfinder: KASENBACHER, Anton, 83278 Traunstein (DE)
(74) Vertreter: Patentanwälte Lambsdorff & Lange
(86) Internationale Anmeldenummer: PCT/EP2009/001250
(87) Internationale Veröffentlichungsnummer: WO 2009/106272

(56) Entgegenhaltungen:
- WO-A-89/01317
- WO-A-90/12548
- WO-A-97/26830
- WO-A-2006/023424
- US-A1- 2005 033 388

## Beschreibung

Die vorliegende Erfindung betrifft ein Laserbearbeitungsgerät zur Bearbeitung von biologischem Gewebe.

Ein beispielhaftes Anwendungsgebiet der vorliegenden Erfindung betrifft das Gebiet der Zahnheilkunde, wobei anstelle eines mechanischen Bohrers zur Ablation bzw. Abtragung von Zahnmaterial, insbesondere kariösem Zahnmaterial, ein Laserbearbeitungs-Verfahren und ein entsprechendes Laserbearbeitungsgerät zum Einsatz kommen können. Die Erfindung kann jedoch ebenso auf andere Arten von biologischem Geweben und Gewebetypen, wie beispielsweise Hartgewebe, Weichgewebe und Gewebeflüssigkeiten, angewendet werden.

In der Zahnheilkunde, insbesondere in der Kariestherapie, besteht ein wesentliches Ziel darin, die konventionelle mechanische Bohrervorrichtung ganz oder teilweise durch einen Laser zu ersetzen. Im Gegensatz zu einer mechanischen Bohrervorrichtung wie einer Turbine, bei der zur Abtragung stets ein mechanischer Kontakt des Bohrers mit dem zu bearbeitenden Bereich vorgesehen ist, ermöglicht ein Laserbearbeitungsgerät eine kontaktlose Bearbeitung und Abtragung. Das Laserbearbeitungsgerät ermöglicht somit eine genauere und insbesondere schmerzlose Operation. Schmerzen können bei dem derzeitigen mechanischen Verfahren insbesondere in der Zahnpulpa entweder durch Vibrationen oder durch Wärmezufuhr ausgelöst werden. Bei der mechanischen Turbine tritt oftmals eine Kombination beider Effekte auf, da sie infolge von Vibration und Rotation auch Reibungswärme erzeugt. Diese Effekte lassen sich beim Einsatz eines Laserbearbeitungsgeräts vermeiden.

Im Laufe der letzten Jahre ist eine Reihe von Lasersystemen im Hinblick auf ihren Einsatz in der Zahnheilkunde untersucht worden. In vielen Fällen und gerade bei den ersten vorgeschlagenen Lasersystemen hat sich jedoch gezeigt, dass entweder unerwünschte thermische oder andere Nebeneffekte auftraten oder dass die Abtragungseffizienz sich als zu gering erwies. Dies gilt insbesondere für Lasersysteme auf der Basis von gepulsten Laserstrahlquellen, die mit Pulsbreiten im Bereich von Nano- bis Mikrosekunden arbeiten, wobei beispielsweise Excimer-Laser mit Wellenlängen im UV-Bereich oder Er: YSGG- (λ = 2,7 µm) oder Er:YAG-Laser (λ = 2,94 µm) im infraroten Wellenlängenbereich verwendet wurden.

Ein wesentlicher Fortschritt ergab sich erst mit dem Einsatz von Kurzpuls-Lasersystemen im Pikosekunden- bzw. Femtosekundenbereich und Wellenlängen im sichtbaren bis nach-infraroten Spektralbereich. In ersten experimentellen Studien konnte bereits gezeigt werden, dass diese Systeme eine qualitativ hochwertige Zahnabtragung ermöglichen, wobei die Abtragungseffizienz zumindest vergleichbar ist mit der Leistungsfähigkeit einer mechanischen Turbine.

Die Druckschrift WO 9 726 830 beschreibt ein Verfahren und eine Vorrichtung zum Abtragen von Material mittels einer gepulsten Laserstrahlquelle. Die zu wählenden Parameter bei der Abtragung hinsichtlich Wellenlänge, Pulsbreite, Energie und Wiederholrate der Laserpulse werden im Wesentlichen lediglich aufgabenhaft derart umschrieben, dass jeder Laserpuls mit einem dünnen Oberflächenbereich des Materials derart wechselwirken soll, dass im Fokus des Bearbeitungs-Laserstrahls ein Plasma gebildet wird. Für die genannten Parameter der gepulsten Laserstrahlung werden relativ weite Bereiche angegeben, wobei insbesondere hinsichtlich der Energie der Laserpulse angegeben wird, dass diese bis zu 50 mJ oder bezogen auf die Fläche bis zu 15 J/cm² betragen kann. Es ist jedoch zu befürchten, dass bei derartig hohen Pulsenergien bei sehr kurzen bis ultrakurzen Laserpulsen die Strahlungsleistung oder -intensität im Pulsmaximum Werte erreicht, bei denen durch nicht-lineare Prozesse wie Multiphotonen-Ionisation, insbesondere unter Beteiligung von mehr als drei Photonen, schädigende Nebeneffekte auftreten. Insbesondere können bei derartigen Pulsspitzenleistungen Wassermoleküle ionisiert werden oder es können DNA-Moleküle ionisiert werden, sodass die Erbsubstanz geschädigt werden kann. Es werden in der Druckschrift keine Angaben dahingehend gemacht, wie derartige Schädigungen sicher vermieden werden können. Insbesondere werden keine diesbezüglich einzustellenden Parameter der gepulsten Laserstrahlung angegeben. Auch aus dem übrigen Stand der Technik sind derartige Parameter nicht bekannt.

Es ist demgemäß Aufgabe der vorliegenden Erfindung, ein Laserbearbeitungsgerät zur Bearbeitung von biologischem Gewebe anzugeben, bei welchen eine effiziente Bearbeitung eines Gewebes gewährleistet werden kann und gleichzeitig schädigende Einflüsse auf das bearbeitete Gewebe und die unmittelbare Umgebung minimiert werden können.

Diese Aufgabe wird mit den Merkmalen der unabhängigen Patentansprüche gelöst. Vorteilhafte Weiterbildungen und Ausgestaltungen sind Gegenstand von Unteransprüchen.

Eine wesentliche Erkenntnis der vorliegenden Erfindung besteht darin, dass ein Laserbearbeitungsgerät zur Bearbeitung von biologischem Gewebe angegeben werden können, welche es erlauben, das Gewebe mit einer ausreichenden Effizienz zu bearbeiten und gleichzeitig dafür Sorge zu tragen, dass im Wesentlichen keine Mehrphotonen-Ionisationsprozesse unter Beteiligung von mehr als drei Photonen stattfinden können. Letzteres bedeutet, dass die in dem Gewebe vorhandenen Wassermoleküle bei der Bearbeitung mit der Laserstrahlung nicht ionisiert, wodurch gesundheitsschädigende Beeinträchtigungen vermieden werden können. Ebenso wird vermieden, dass durch die genannten Mehrphotonen-Ionisationsprozesse unter Beteiligung von mehr als drei Photonen DNA-Moleküle ionisiert werden und somit eine Schädigung der Erbsubstanz verursacht wird. Die Erfindung erreicht dies durch Angabe eines Satzes von Parametern eines gepulsten Bearbeitungs-Laserstrahls.

Gemäß die Erfindung wird ein Laserbearbeitungsgerät zur Bearbeitung von biologischem Gewebe angegeben, welches eine Laserstrahlquelle zum Bereitstellen eines gepulsten Bearbeitungs-Laserstrahls und ein Fokussiermittel zum Fokussieren des Bearbeitungs-Laserstrahls umfasst, wobei die Laserstrahlquelle eingerichtet ist, um eine Wellenlänge der Laserpulse in einem Bereich zwischen 700 nm und 1400 nm einzustellen, eine zeitliche Halbwertsbreite der Laserpulse in einem Bereich zwischen 5 ps und 100 ps einzustellen, und eine Energiedichte der Laserpulse in einem Bereich zwischen 1,5 J/cm² und 7,5 J/cm² einzustellen.

Gemäß die Erfindung kann das Laserbearbeitungsgerät als ein dentales Laserbearbeitungsgerät zur Ablation oder Abtragung von Zahnmaterial, insbesondere kariösem Zahnmaterial, ausgebildet sein.

Gemäß einer weiteren Ausführungsform kann das Laserbearbeitungsgerät des Weiteren eine Strahlformungs-Einheit zur Formung eines im Wesentlichen rechteckförmigen (tophat) Strahlprofils aufweisen.

Gemäß einer weiteren Ausführungsform kann das Laserbearbeitungsgerät des Weiteren eine Scan-Einheit zum Abrastern oder Scannen eines Bereichs des Gewebes mit dem Bearbeitungs-Laserstrahl aufweisen. Die Scan-Einheit kann derart ausgelegt sein und insbesondere eine derartige Scan-Geschwindigkeit aufweisen, dass ein von dem Fokus des Bearbeitungs-Laserstrahls erfasster Teilbereich von genau einem Laserpuls beaufschlagt wird. Die Scan-Einheit kann ferner derart ausgelegt sein, dass einander benachbarte und von je einem Laserpuls erfasste Teilbereiche einen räumlichen Überlapp miteinander haben, dessen Fläche kleiner als die Hälfte oder kleiner als ein anderer Bruchteil eines Teilbereichs ist.

Gemäß einer weiteren Ausführungsform kann das Laserbearbeitungsgerät ferner eine Autofokus-Einheit zum Konstanthalten der räumlichen Lage des Fokus auf der Oberfläche des Gewebes aufweisen.

Es kann dabei noch zusätzlich vorgesehen sein, dass eine möglichst grosse numerische Apertur (NA) gewählt wird, dass also unter den gegebenen Möglichkeiten für die Anordnung und Ausgestaltung der Linse und der Autofokuseinheit diejenige mit der grösstmöglichen numerischen Apertur ausgewählt wird. Je grösser die numerische Apertur ist, umso kleiner ist die erreichbare Fokusgrösse (oder das Fokusvolumen) auf der zu bearbeitenden Gewebeoberfläche und umso kleiner kann die Energie der Laserpulse gewählt werden.

Gemäß die Erfindung hot das Laserbearbeitungsgerät ferner eine Detektions-Einheit zur Detektion des Vorhandenseins eines in dem Gewebe oder in einer Umgebung davon erzeugten Signals und gegebenenfalls von dessen Signalstärke aufweisen. Mit der Detektions-Einheit kann eine Kontrolleinheit verbunden sein, welche zum Ein- oder Ausschalten der Laserstrahlquelle in Abhängigkeit von einem von der Detektions-Einheit gelieferten Signal ausgelegt ist.

Die Detektions-Einheit weist einen optischen Sensor auf, welcher beispielsweise für die Erfassung einer Strahlung eines bei der Bearbeitung erzeugten Plasmas oder für die Erfassung einer zweiten oder höheren Harmonischen einer auf das Gewebe eingestrahlten elektromagnetischen Strahlung ausgelegt ist.

Die Detektions-Einheit kann auch einen akustischen Sensor aufweisen, falls das zu detektierende Signal ein akustisches Signal ist.

Die Kontrolleinheit kann dafür ausgelegt sein, die Laserstrahlquelle auf einen Bearbeitungs-Modus oder einen Diagnose-Modus einzustellen, wobei im Bearbeitungs-Modus der gepulste Bearbeitungs-Laserstrahl erzeugt wird und im Diagnose-Modus ein insbesondere gepulster Diagnose-Laserstrahl erzeugt wird, mit dem eine Bearbeitung des Gewebes nicht möglich ist und/oder dessen Laserpulse eine Energiedichte aufweisen, welche kleiner ist als die Energiedichte, welche zur Bearbeitung des Gewebes erforderlich ist.

Gemäß einer weiteren Ausführungsform kann das Laserbearbeitungsgerät ferner, insbesondere wenn es als ein dentales Laserbearbeitungsgerät ausgelegt ist, ein Fixiermittel aufweisen, mit welchem eine als ein Handstück ausgebildete Auskoppeleinheit mit ihrem distalen Ende in Bezug auf den zu bearbeitenden Zahn fixiert wird.

Im Folgenden werden Ausführungsbeispiele näher erläutert. Es zeigen:
- Fig. 1: eine schematische Darstellung einer Ausführungsform eines Laserbearbeitungsgeräts;
- Fig. 2: eine schematische Darstellung einer weiteren Ausführungsform eines Laserbearbeitungsgeräts; und
- Fig. 3A, B: schematische Darstellungen zweier Ausführungsformen eines mit einem distalen Ende einer LaserstrahlAuskoppeleinheit verbundenen Fixierelements.

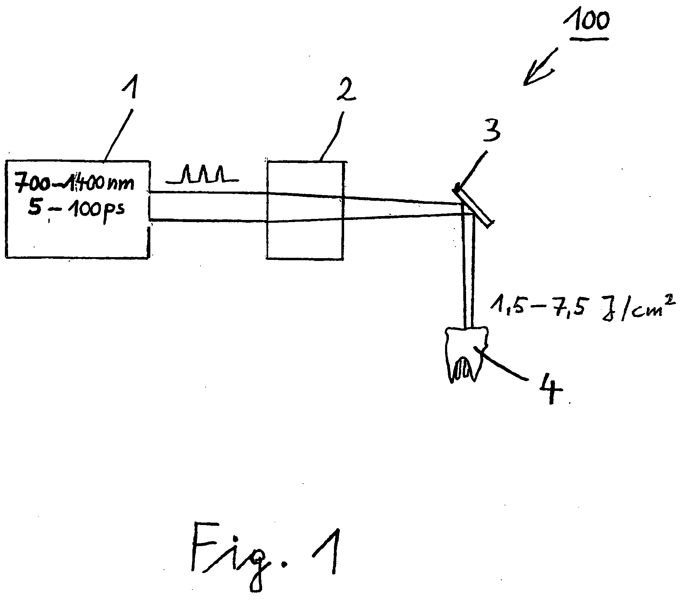

In der Fig. 1 ist eine Ausführungsform eines Laserbearbeitungsgeräts schematisch dargestellt. Das Laserbearbeitungsgerät 100 ist im dargestellten Fall ein dentales Laserbearbeitungsgerät, mit welchem Zahnmaterial, insbesondere kariöses Zahnmaterial, bearbeitet bzw. abgetragen oder ablatiert werden kann. Das Laserbearbeitungsgerät kann jedoch auch ein anderes medizinisches Laserbearbeitungsgerät zur Bearbeitung einer anderen Art von biologischem Gewebe sein.

Das Laserbearbeitungsgerät 100 weist eine Laserstrahlquelle 1 auf, welche einen gepulsten Bearbeitungs-Laserstrahl emittiert. Die Wellenlänge des Bearbeitungs-Laserstrahls liegt in einem Bereich zwischen 700 nm und 1400 nm, während die Pulsdauer der Laserpulse in einem Bereich zwischen 5 ps und 100 ps liegt.

Der Bearbeitungs-Laserstrahl wird einer Fokussiereinheit 2 zugeführt, mittels welcher der Bearbeitungs-Laserstrahl auf einen zu bearbeitenden Zahn 4 eines Patienten fokussiert wird. Gegebenenfalls kann der Bearbeitungs-Laserstrahl vorher durch eine optische Umlenkeinheit 3 wie einen Spiegel oder ein Umlenkprisma umgelenkt werden. Der Bearbeitungs-Laserstrahl wird derart fokussiert, dass die Laserpulse auf der Oberfläche des Zahns 4 eine Energiedichte in einem Bereich zwischen 1,5 J/cm² und 7,5 J/cm² besitzen.

Es kann dabei vorgesehen sein, dass die Laserstrahlquelle 1 die Laserpulse solchermaßen generiert, dass diese eine Energie pro Puls in einem Bereich unterhalb von 100 µJ aufweisen. In diesem Fall ist die Fokussiereinheit 2 für die Einhaltung der obigen Werte für die Energiedichte derart einzustellen, dass der Bearbeitungs-Laserstrahl auf der Oberfläche des Zahns 4 einen Fokus mit einem Fokusdurchmesser in einem Bereich von 10 µm bis 100 µm aufweist.

Es kann weiter vorgesehen sein, dass die Laserstrahlquelle 10 die Laserpulse mit einer Repetitionsrate in einem Bereich von 500 Hz bis 1000 kHz emittiert.

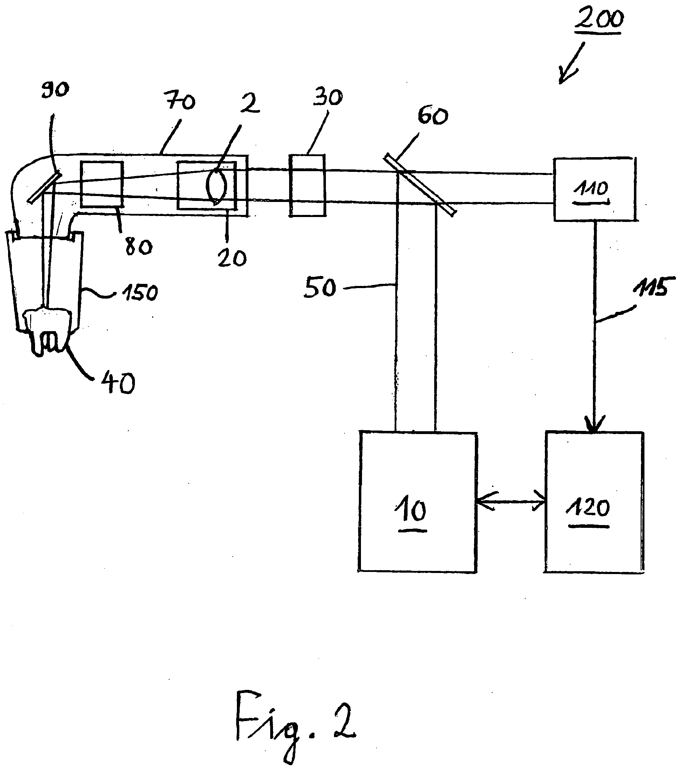

In der Fig. 2 ist eine weitere Ausführungsform eines Laserbearbeitungsgeräts schematisch und mit nicht maßstabgetreuen Größenverhältnissen dargestellt. Die in der Fig. 2 gezeigte Ausführungsform eines Laserbearbeitungsgeräts 200 umfasst eine Laserstrahlquelle 10, die einen gepulsten Bearbeitungs-Laserstrahl 50 emittiert. Die Laserstrahlquelle 10 ist in dem gezeigten Ausführungsbeispiel ein mit einem transienten oder einem regenerativen Verstärker gekoppelter Nd:YAG-Laser, welcher Laserpulse bei einer Wellenlänge von 1064 nm emittiert. Es kann hier auch eine andere Laserstrahlquelle wie beispielsweise ein Nd:YVO₄- oder ein Nd:GdVO₄-Laser verwendet werden. Die Pulsdauer der Laserpulse beträgt 10 ps und die Wiederholrate der Laserpulse liegt in einem Bereich zwischen 1 kHz und 100 kHz. Die Energie der Laserpulse beträgt 40 µJ. Bei einer Repetitionsrate von 100 kHz beträgt die mittlere Strahlungsleistung 4 W.

Der von der Laserstrahlquelle 10 emittierte Bearbeitungs-Laserstrahl 50 trifft in seinem Strahlenverlauf bei der in der Fig. 2 gezeigten Ausführungsform auf eine optische Umlenkeinheit 60, die jedoch nur für die Wellenlänge des Bearbeitungs-Laserstrahls 50 als Umlenkeinheit wirkt, sodass der Bearbeitungs-Laserstrahl 50 um ca. 90° umgelenkt wird.

Im Strahlengang des Bearbeitungs-Laserstrahls 50 befindet sich anschliessend eine Strahlformungs-Einheit 30, mit welcher ein rechteckförmiges oder Tophat-Strahlprofil erzeugt wird.

Anschließend tritt der Bearbeitungs-Laserstrahl 50 in eine als ein Handstück ausgebildete Laserstrahl-Auskoppeleinheit 70 ein. Die Auskoppeleinheit 70 enthält in ihrem vorderen Abschnitt eine Linse 2, die Teil einer Autofokus-Einheit 20 ist. Die Autofokus-Einheit 20 bewirkt mit an sich bekannten Mitteln, dass der von der Linse 2 erzeugte Fokus stets innerhalb der Ebene der bearbeiteten Oberfläche des Zahns 40 liegt. Die Autofokus-Einheit 20 kann insbesondere mit einer optischen Sensoreinrichtung zusammenwirken, welche eine von der Oberfläche des Zahns zurückgeworfene Strahlung dahingehend auswertet, ob die Oberfläche noch im Fokus der Laserstrahlung liegt. Darauf hin wird ein Steuersignal an die Autofokus-Einheit 20 übermittelt, um eine geeignete Maßnahme zu ergreifen, so dass die Oberfläche des Zahns 40 wieder in den Fokus des Laserstrahls gelangt. Diese Maßnahme kann beispielsweise in einer Bewegung der Linse 2 vor oder zurück entlang des Ausbreitungsweges des Laserstrahls 50 bestehen. Dies kann durch einen schnellen Schrittmotor gewährleistet werden, der mit einem Schlitten verbunden ist, auf welchem die Linse 2 montiert ist. Es kann aber auch vorgesehen sein, dass die Linse 2 derart ausgestaltet ist, dass sich ihre Brechkraft verändern lässt.

Gemäß der Ausführungsform der Fig. 2 ist die Linse 2 derart angeordnet, dass sie auf der Oberfläche des Zahns 40 einen Fokus mit einem Fokusdurchmesser von 40 µm erzeugt. Mit dem weiter oben genannten Wert für die Pulsenergie eines Laserpulses ergibt sich somit eine Energiedichte von 3,18 J/cm², woraus sich wiederum eine Pulsspitzenintensität von 3,18 - 1011 W/cm² ergibt. Dies entspricht einer Photonen-Flussdichte von 1,7 · 10³⁰ Photonen ·cm⁻² · s⁻¹. Die elektrische Feldstärke des elektromagnetischen Wechselfeldes beträgt 1,55 · 10⁷ V/cm und die mittlere Oszillationsenergie der Elektronen im elektromagnetischen Wechselfeld beträgt 0,021 eV.

Es sei angemerkt, dass die Strahlformungseinheit 30 sich auch im Strahlengang hinter der Linse 2, also insbesondere auch im Handstück 70 befinden kann. Es kann auch vorgesehen sein, dass Autofokuseinheit 20 und Strahlformungseinheit 30, insbesondere Linse 2 und Strahlformungseinheit 30, zu einem gemeinsamen optischen Bauelement zusammengefasst werden.

In einem weiteren Abschnitt der Auskoppeleinheit 70 ist eine Scan-Einheit 80 angeordnet, mit welcher der Bearbeitungs-Laserstrahl 50 in ebenfalls an sich bekannter Weise, beispielsweise mittels zweiter sich gegenüberstehender rotierender Spiegel, über einen bestimmten Bereich der zu bearbeitenden Oberfläche des Zahns 40 gerastert oder gescannt werden kann. Mittels einer weiteren Umlenkeinheit 90, wie beispielsweise eines Umlenkprismas oder eines reflektierenden Spiegels wird dann der Bearbeitungs-Laserstrahl 50 in Richtung auf den Zahn 40 umgelenkt.

In dieser Ausführungsform ist also die Scan-Einheit 80 im Handstück angeordnet. Es sind jedoch auch andere Ausführungsformen denkbar, in denen die Scan-Einheit im Strahlengang vor dem Handstück, d.h. insbesondere innerhalb eines Spiegelgelenkarms oder am Eingang eines Spiegelgelenkarms der optischen Einrichtung vor dem Handstück angeordnet ist.

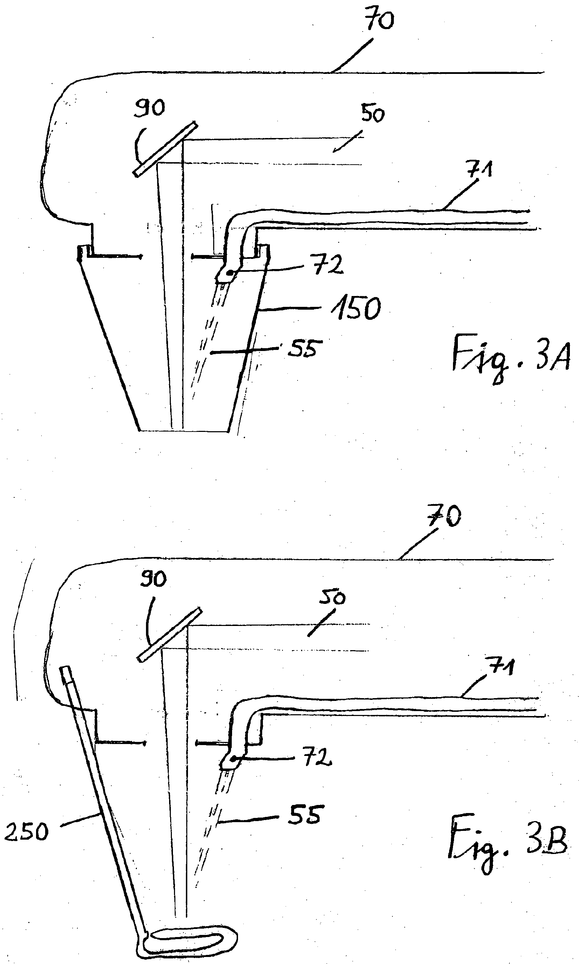

Die als ein Handstück ausgebildete Auskoppeleinheit 70 muss bei der Bearbeitung von dem behandelnden Arzt gehalten und auf den zu bearbeitenden Zahn gerichtet werden. Um diese Art von Handhabung zu erleichtern und insbesondere um die Position und Ausrichtung des distalen Endes der Auskoppeleinheit 70 in Bezug auf den zu bearbeitenden Zahn 40 konstant zu halten, ist ein trichterförmiges Fixierelement 150 an dem distalen Ende der Auskoppeleinheit 70 befestigt und kann während der Bearbeitung an dem Zahn 40 geeignet fixiert werden, wie in Verbindung mit Fig. 3A noch näher erläutert werden wird.

Das Behandlungsverfahren kann zusätzlich unter einer vorgegebenen kontrollierten Atmosphäre oder unter anderen vorgegebenen Bedingungen wie Airflow oder dergleichen durchgeführt werden.

Die in dem bearbeiteten Bereich der Oberfläche des Zahns 40 oder in einer Umgebung davon erzeugten optischen oder akustischen Signale können detektiert und zu Diagnosezwecken verwendet werden. Bezüglich optischer Signale wurde bereits erläutert, dass diese beispielsweise entweder auf der Plasmastrahlung oder der zweiten (SHG, second harmonic generation) oder einer höheren Harmonischen von elektromagnetischer Strahlung beruhen, die auf das für eine Bearbeitung vorgesehene Zahnmaterial einwirkt. Das in der Fig. 2 gezeigte Ausführungsbeispiel soll im Folgenden anhand des letztgenannten Falls der Detektion eines SHG-Signals erläutert werden.

Bei dieser Art der Diagnose wird in der Laserstrahlquelle 10 ein Diagnose-Modus eingestellt, bei welchem ein Diagnose-Laserstrahl emittiert wird, dessen Energie oder Energiedichte unterhalb der Ablations- oder Plasma-Erzeugungsschwelle liegt, sodass mit diesem Laserstrahl keine Bearbeitung stattfinden kann. Mit dem Diagnose-Laserstrahl, welcher ebenso wie der Bearbeitungs-Laserstrahl gepulst ist, soll untersucht werden, ob ein zu bearbeitender Oberflächenbereich des Zahns gesundes oder kariöses Zahnmaterial enthält. Gesundes Zahnmaterial liefert ein höheres SHG-Signal als kariöses Material. Die solchermaßen an der Zahnoberfläche erzeugte frequenzverdoppelte Strahlung durchläuft in umgekehrter Richtung zumindest teilweise den Strahlengang des Bearbeitungs-Laserstrahls, wie er oben beschrieben worden ist, wird also von der Umlenkeinheit 90 umgelenkt, durchläuft die Scan-Einheit 80, die Autofokus-Einheit 20 mit der Linse 2 und trifft schließlich auf den Strahlteiler 60, der jedoch für die Wellenlänge des SHG-Signals durchlässig ist, sodass die frequenzverdoppelte Strahlung einer optischen Erfassungseinrichtung 110 zugeführt werden kann. Als optische Erfassungseinrichtung 110 kann ein einfacher Photodetektor vorgesehen sein, mit dem die Intensität der SHG-Strahlung gemessen wird. Es kann auch vorgesehen sein, dass als optische Erfassungseinrichtung 110 ein komplexeres System wie ein Spektrometer, eine CCD-Kamera oder ein CMOS-Bildsensor verwendet wird. Derartige optische Erfassungseinrichtungen können beispielsweise gleichzeitig dafür dienen, mit der Autofokus-Einheit 20, wie oben bereits angedeutet, in geeigneter Weise zusammenzuwirken.

Die von der optischen Erfassungseinrichtung 110 ermittelten Werte für die SHG-Strahlung werden in ein Signal 115 umgewandelt und einer kombinierten Auswerte- und Steuereinheit 120 zugeführt, wobei es sich bei der Auswerte- und Steuereinheit 120 bei dieser Ausführungsform auch um ein Computersystem handeln kann. Es sind aber auch prinzipiell alle anderen Steuer- und Regelsysteme denkbar, wie z.B. SPS (speicherprogrammierbare Steuerungen), Mikro-Controller oder analoge Regelkreise.

Der Auswerte- und Steuereinheit 120 kann ein Signal von der Laserstrahlungsquelle 10 zugeführt werden, welches Daten über den Betriebszustand der Laserstrahlungsquelle 10 enthält. Die Auswerte- und Steuereinheit 120 gibt in Abhängigkeit von dem von der optischen Erfassungseinrichtung 110 übermittelten Signal 115 ein Steuersignal aus, welches der Laserstrahlungsquelle 10 zugeführt wird und die Laserstrahlungsquelle 10 beispielsweise von einem Ruhemodus in einen Bearbeitungsmodus schaltet, etwa wenn die optische Erfassungseinrichtung 110 aufgrund eines zu niedrigen, einen kariösen Zustand anzeigendes SHG-Signals ein entsprechendes Signal 115 der Auswerte- und Steuereinheit 120 übermittelt.

Die in der Fig. 2 gezeigte Ausführungsform umfasst insbesondere eine Laserstrahlungsquelle 10, die schnell zwischen den Betriebsarten "Aus" (Ruhemodus), "Diagnostik" und "Therapie" (Bearbeitung) umschalten kann. Hierbei soll unter "schnell" eine Zeit verstanden werden, die sowohl von dem Patienten als auch von dem behandelnden Arzt nicht als Unterbrechung der Behandlung wahrgenommen wird, d.h. beispielsweise kleiner als 0,1 Sek.

In dieser Ausführungsform emittiert die Laserstrahlquelle 10 sowohl den Bearbeitungs-Laserstrahl während dem "Therapie"-Modus als auch unter geänderten Bedingungen den Diagnose-Laserstrahl während dem "Diagnostik"-Modus. Der Unterschied zwischen dem Diagnostik- und dem Therapie-Modus ist im Wesentlichen die Energiedichte pro Puls in J/cm², die auf die Zahnoberfläche appliziert wird. Im Diagnose-Modus muss die Energiedichte sicher unterhalb der Ablations-Schwelle sein, während sie im Therapie-Modus darüber liegt.

Unterschieden werden kann zwischen Methoden des schnellen Umschaltens innerhalb und außerhalb des Lasers. Zum Schalten werden üblicherweise akusto-optische oder elektro-optische Modulatoren eingesetzt. Bei der akusto-optischen Modulation wird der Laserstrahl räumlich durch die Beugung in einem Brechungsindexgitter abgelenkt, im Fall der elektro-optischen Modulation erfolgt eine Polarisationsdrehung, die zusammen mit einem polarisationsselektiven optischen Element (Polarisator) die Modulation hervorruft. Bei den optischen Verstärkern unterscheidet man zwischen transienten Einfach- oder Mehrfach-Durchlaufverstärkern und den regenerativen Verstärkern, die einen Verstärkerresonator besitzen.

Die verwendete Laserstrahlquelle kann beispielsweise einen transienten Verstärker enthalten und ein elektro-optischer Modulator (EOM) kann zwischen dem ps-Oszillator und dem Verstärker angeordnet sein. Die Schalttechnik erlaubt ein An- und Ausschalten der Hochspannung des EOM (beispielsweise Pokkels-Zelle) mit Schaltzeiten von 4 - 5 ns. Außerdem kann das System auch Bursts von Laserpulsen erzeugen, d.h. mehr als einen verstärkten ps-Impuls pro Schaltzyklus.

Damit stehen für das schnelle Umschalten zwischen den Betriebsarten die folgenden Varianten zur Verfügung:
A) Umschalten der Repetitionsrate
   Mit dem Umschalten der Repetitionsrate wird die Pulsenergie pro Puls verändert. Höhere Repetitionsraten haben bedingt durch die Funktionsweise des Lasers geringere Pulsenergien als niedrigere Repetitionsraten. Der Therapie-Modus weist dann eine niedrige Repetitionsrate von beispielsweise 100 kHz auf, während der Diagnostik-Modus bei einer hohen Repetitionsrate von beispielsweise 500 kHz läuft, wobei der Energieunterschied der Pulsenergien zwischen den beiden Modi einen bestimmten Faktor betragen kann, der von dem Repetitionsratenhub und der verwendeten Laserstrahlquelle abhängen kann. Dieser Faktor kann beispielsweise eine Zahl zwischen 1 und 10, beispielsweise 3 oder 5, oder auch eine andere Zahl sein. Dieses Umschalten geschieht prinzipiell pulsgenau, d.h. von einem Puls zum nächsten, und die tatsächliche Geschwindigkeit hängt von der Signalverarbeitung der Software und der Elektronik ab, die typisch bei 100 ns liegt. Das Umschalten der Repetitionsrate kann innerhalb einer Zeit kürzer als 1 ms oder sogar kleiner als 1 µs erfolgen.
B) Umschalten von Single-Puls-Betrieb auf Burst-Mode Hier wird bei fester Repetitionsrate von beispielsweise 100 kHz die Energie des Laserimpulses im Single-Puls-Betrieb auf mehrere Impulse verteilt, sodass die Pulsenergie der Pulse im Burst kleiner als die Ablations-Schwelle ist, sodass also im Therapie-Modus der Single-Puls-Betrieb eingestellt wird, während im Diagnostik-Modus der Burst-Betrieb eingestellt wird. Dieses Umschalten geschieht wie im Fall A) prinzipiell pulsgenau und ist ebenso durch die Signalverarbeitung der Software und Elektronik limitiert. Somit kann das Umschalten ebenfalls schneller als 1 µs erfolgen.
C) An- und Ausschaltung der Hochspannung für den EOM
   Diese Methode erlaubt bei den besten heutigen HV-Netzteilen Schaltzeiten von > 1 ms. Nachteilig kann gegebenenfalls bei derart schnellem Schalten eine Abweichung von dem idealen Rechteck-Impuls sein, das sich optisch in unterschiedlich starken Pulsen wiederspiegeln würde. Außerdem kommt es beim Einschalten durch die gespeicherte Pumpenergie im Verstärker zu einer Erhöhung der Pulsenergie der ersten Pulse. Diese Pulsenergie-Überhöhung kann abhängig von der Auslegung des Verstärkers bis zu einer Größenordnung betragen.
D) Motorische Drehung der Polarisation
   Zusammen mit einem polarisationsselektiven Element kann die Energie der Laserpulse gesteuert werden. Dieses ist in den Standard-Lasern der Anmelderin Lumera implementiert. Es wird erwartet, dass mit verbesserten Motoren oder einem schnellen Hin- und Herklappen Umschaltzeiten bis etwa 10 ms erreicht werden können.

Der ps-Laser kann auch im konstanten Modus betrieben werden und die Modulation der Ausgangsstrahlung kann mit weiteren externen Modulatoren (AOM, EOM), die dann frei triggerbar sind, realisiert werden. Die Geschwindigkeit bei Verwendung eines externen EOMs entspricht der vom Fall A) und B), während man im Fall C) bei externer Modulation nicht den Nachteil der Energie-Überhöhung der ersten Impulse hat.

Es wird angemerkt, dass in der Umschaltbarkeit der Laserstrahlquelle zwischen einem Therapie- oder Bearbeitungs-Modus und einem Diagnostik-Modus bei geänderten Parametern der Laserstrahlung eine eigenständige Erfindung gesehen wird, die unabhängig von dem Hauptaspekt der vorliegenden Anmeldung, nämlich den Parametern des gepulsten Bearbeitungs-Laserstrahls, ist. Dieser Aspekt bezieht sich somit auf ein LaserbearbeitUngsgerät zur Bearbeitung von biologischem Gewebe, welches eine Laserstrahlquelle zum Bereitstellen eines gepulsten Bearbeitungs-Laserstrahls und eines gepulsten Diagnose-Laserstrahls, umfasst, wobei die Laserpulse des gepulsten Diagnose-Laserstrahls eine Pulsenergie aufweisen, die geringer ist als die zur Bearbeitung bzw. Ablation des Gewebes notwendige Pulsenergie. Das Laserbearbeitungsgerät kann optional ein oder mehrere weitere Merkmale aufweisen, wie sie in dieser Anmeldung in Verbindung mit dem Hauptaspekt beschrieben wurden.

In einer weiteren nicht zeichnerisch dargestellten Ausführungsform kann auch vorgesehen sein, dass als Detektionssignal ein akustisches Signal herangezogen wird. Es wurde festgestellt, dass bei der Erzeugung eines Plasmas während des Bearbeitungsmodus ein charakteristisches akustisches Signal erzeugt wird. Sowie die Plasmastrahlung durch einen optischen Sensor detektiert werden kann, kann analog das akustische Geräusch durch einen akustischen Sensor erfasst werden und der Auswerte- und Steuereinheit 120 zur weiteren Verarbeitung zugeführt werden. Es wird angemerkt, dass in der Detektion des Vorhandenseins eines akustischen Signals und gegebenenfalls von dessen Signalstärke eine eigenständige Erfindung gesehen wird. Dieser Aspekt bezieht sich somit auf ein Laserbearbeitungsgerät zur Bearbeitung von biologischem Gewebe, welches eine Laserstrahlquelle zum Bereitstellen eines gepulsten Bearbeitungs-Laserstrahls, und eine Detektions-Einheit zur Detektion eines akustischen Signals und zur Steuerung der Laserstrahlquelle in Abhängigkeit von dem Vorhandensein und gegebenenfalls der Signalstärke des akustischen Signals umfasst. Das Laserbearbeitungsgerät kann optional ein oder mehrere weitere Merkmale aufweisen, wie sie in dieser Anmeldung in Verbindung mit dem Hauptaspekt beschrieben wurden.

In den Fig. 3A,B sind Ausführungsbeispiele für ein Fixierelement perspektivisch in einem etwas grösseren Detail dargestellt. In dem Ausführungsbeispiel der Fig.3A ist das Fixierelement 150 als Trichter ausgebildet. Das trichterförmige Fixierelement 150 kann beispielsweise als Aufsatz ausgebildet sein, welcher an einem distalen Ende der als Handstück ausgebildeten Auskoppeleinheit 70 in geeigneter Weise befestigt, z.B. aufgeschraubt werden kann. Je nach der Bearbeitungssituation kann ein Aufsatz mit einer gewünschten Länge oder Form oder einem geeigneten Durchmesser am unteren Ende ausgewählt werden. Er kann wie dargestellt einen sich nach unten verjüngenden Durchmesser aufweisen. Es sind jedoch auch Formen denkbar, deren Durchmesser sich nach unten verbreitern. Die trichterförmige Form des Fixierelements bietet ausser der Fixierung die Möglichkeit, die Behandlung in einem von dem übrigen Mundraum abgeschlossenen Raum durchführen zu können. Des Weiteren kann das ablatierte Material kontrolliert abgesaugt und durch das Handstück abgeführt werden (nicht dargestellt). Des Weiteren kann ein weiteres Medium 55 wie Kühlungsluft durch eine im Handstück geführte Leitung 71 zugeführt und durch eine untere Öffnung des Handstücks in Richtung auf den behandelten Zahn gelenkt werden. An der Öffnung kann zu diesem Zweck eine verstellbare Düse 72 angebracht sein, durch die das Medium 55 gezielt auf den behandelten Bereich gelenkt wird.

In einer weiteren Ausführungsform können mikroskopische Partikel wie Nanopartikel oder dergleichen auf den behandelten Bereich gerichtet werden. Diese Nanopartikel können eine bestimmte vorgegebene Form aufweisen, um eine gewünschte Wechselwirkung mit dem ablatierten oder mit einem noch nicht ablatierten und für eine Bearbeitung vorgesehenen Material einzugehen, und damit den gesamten Bearbeitungsprozess und insbesondere den Ablationsprozess günstig zu beeinflussen. Beispielsweise können die Nanopartikel zur Gewebsdifferenzierung (gesund/erkrankt) eingesetzt werden. Auch können die Nanopartikel in Verbindung mit Verfahren wie photodynamische Therapie (PDT), Photopolymerisation (beispielsweise Abdeckung einer offenen Pulpa per Verbund synthetischer kollagener Fasern) oder Photokoagulation durch ihre Wechselwirkung mit dem Gewebe günstige und den Prozess fördernde Wirkungen zeitigen. Eine weitere vorteilhafte Wirkung von Nanopartikeln könnte in einer Steigerung der Ablationseffizienz bei unveränderter Laserintensität oder umgekehrt in einer Minimierung der Laserintensität bei gleichbleibender Ablationseffizienz (je nach Priorität) bestehen. Da stets oberste Maxime des behandelnden Arztes eine Minimierung der schädigenden Begleiterscheinungen (Prinzip des primum non nocere) ist, können Nanopartikel auch dazu genutzt werden, noch vorhandene freie Elektronen weiter zu reduzieren, um so die biologischmedizinische Verträglichkeit (Biosafety) weiter zu steigern.

Die mikroskopischen Partikel können beispielsweise eine Grösse von 1 nm bis 1 µm aufweisen. Sie können zusammen mit einem Medium 55 wie Kühlungsluft zugeführt werden und beispielsweise als angereicherte Kühlungsluft durch die Zuführungsleitung 71 zugeführt und mittels der Düse 72 gezielt auf den behandelten Bereich gerichtet werden.

Es sei angemerkt, dass auch in der Zuführung von mikroskopischen Partikeln wie Nanopartikeln bei der Laserbearbeitung von biologischem Gewebe eine eigenständige Erfindung gesehen wird, die unabhängig von dem Hauptaspekt der vorliegenden Anmeldung, nämlich den Parametern des gepulsten Bearbeitungs-Laserstrahls, ist.

In dem Ausführungsbeispiel der Fig.3B ist das Fixierelement als Drahtbügel 250 ausgebildet. Der Drahtbügel 250 ist an einem distalen Ende der als Handstück ausgebildeten Auskoppeleinheit 70 in geeigneter Weise befestigt, etwa indem er beispielsweise durch eine Öffnung in der Außenwand des Handstücks geführt ist. Er besteht im Wesentlichen aus einem einstückigen Draht, welcher einen langgezogenen linearen Abschnitt aufweist, welcher an seinem unteren Ende in einen weiteren Abschnitt mündet, der zu einem Ring geformt ist. Der ringförmige Abschnitt ist dabei nicht vollständig in sich geschlossen, vielmehr endet ein Ende des ringförmigen Abschnitts in einem Abstand von dem Punkt, an welchem der lineare Abschnitt in den ringförmigen Abschnitt übergeht. Somit kann der ringförmige Abschnitt in gewissen Grenzen in seinem Durchmesser verändert werden. Der ringförmige Abschnitt dient dazu, während der Bearbeitung den zu bearbeitenden Zahn zu umfassen und solchermaßen für eine fixe Positionierung und Ausrichtung des distalen Endabschnitts des Handstücks in Bezug auf den zu bearbeitenden Zahn zu sorgen.

Es wird angemerkt, dass in der generellen Anordnung eines Fixierelements zur Fixierung der räumlichen Position eines Handstücks für die Laserbearbeitung von biologischem Gewebe in Bezug auf einen zu bearbeitenden Bereich eine eigenständige Erfindung gesehen wird, die unabhängig von dem Hauptaspekt der vorliegenden Anmeldung, nämlich den Parametern des gepulsten Bearbeitungs-Laserstrahls, ist. Dieser Aspekt bezieht sich somit auf ein Fixierelement, welches zwischen einer Laserstrahl-Auskoppeleinheit und einem Abschnitt eines zu bearbeitenden biologischen Gewebes befestigbar ist. Das Fixierelement kann optional ein oder mehrere weitere Merkmale aufweisen, wie sie in dieser Anmeldung beschrieben wurden. Dieser Aspekt kann sich auch auf eine ein derartiges Fixierelement enthaltende, insbesondere einstückig mit dem Fixierelement verbundene Laserstrahl-Auskoppeleinheit beziehen.

## Patentansprüche

1. Dentales Laserablationsgerät zur Ablation oder Abtragung von Zahnmaterial, insbesondere kariösem Zahnmaterial , umfassend:
eine Laserstrahlquelle (1; 10) zum Bereitstellen eines gepulsten Ablations-Laserstrahls (50), und
ein Fokussiermittel (2) zum Fokussieren des Ablations-Laserstrahls (50), wobei der Ablations-Laserstrahl (50) aufweist:
eine Wellenlänge der Laserpulse in einem Bereich zwischen 700 nm und 1400 nm,
eine zeitliche Halbwerts-Breite der Laserpulse in einem Bereich zwischen 5 ps und 100 ps, und
eine Energiedichte der Laserpulse auf der Oberfläche eines Zahns in einem Bereich zwischen 1,5 J/cm² und 7,5 J/cm²,
**gekennzeichnet durch**
eine Detektions-Einheit (110) zur Detektion des Vorhandenseins eines in dem Zahn oder in einer Umgebung davon erzeugten Signals und gegebenenfalls von dessen Signalstärke, wobei die Detektions-Einheit (110) einen optischen Sensor aufweist und **dadurch** gekennzeichnet, dass der optische Sensor für die Erfassung einer zweiten oder höheren Harmonischen einer auf den Zahn eingestrahlten elektromagnetischen Strahlung ausgelegt ist.

2. Laserablationsgerät nach Anspruch 1, bei welchem eine Energie der Laserpulse in einem Bereich unterhalb von 100 µJ eingestellt ist, und
ein Fokus des Ablations-Laserstrahls (50) auf einer Oberfläche des Zahns mit einem Fokusdurchmesser in einem Bereich zwischen 10 µm und 100 µm eingestellt ist.

3. Laserablationsgerät nach Anspruch 1 oder 2, bei welchem eine Wiederholrate der Laserpulse in einem Bereich zwischen 500 Hz und 1000 kHz eingestellt ist.

4. Laserablationsgerät nach einem der Ansprüche 1 bis 3, ferner umfassend:
eine Laserstrahl-Auskoppeleinheit (70), und
ein mit der Laserstrahl-Auskoppeleinheit (70) verbundenes Fixiermittel (150) zur räumlichen Fixierung eines distalen Endes der Laserstrahl-Auskoppeleinheit (70) in Bezug auf einen Abschnitt des zu ablatierenden Zahns.

5. Laserablationsgerät nach einem der Ansprüche 1 bis 4, ferner umfassend:
eine Strahlformungs-Einheit (30) zur Formung eines im Wesentlichen rechteckförmigen Strahlprofil des gepulsten Ablations-Laserstrahls (50).

6. Laserablationsgerät nach einem der Ansprüche 1 bis 5, ferner umfassend:
eine Scan-Einheit (80) zum Abrastern oder Scannen eines Bereichs des Zahns mit dem Ablations-Laserstrahl (50).

7. Laserablationsgerät nach Anspruch 6, bei welchem die Scan-Einheit (80) derart ausgelegt ist, dass ein von dem Fokus des Ablations-Laserstrahls (50) erfasster Teilbereich von genau einem Laserpuls beaufschlagt wird.

8. Laserablationsgerät nach Anspruch 6 oder 7, bei welchem die Scan-Einheit (80) derart ausgelegt ist, dass einander benachbarte und von je einem Laserpuls erfasste Teilbereiche einen räumlichen Überlapp miteinander haben, dessen Fläche kleiner als die Hälfte eines Teilbereichs ist.

9. Laserablationsgerät nach einem der Ansprüche 1 bis 8, ferner umfassend:
eine Autofokus-Einheit (20) zum Konstanthalten der räumlichen Lage des Fokus des Ablations-Laserstrahls (50) auf der Oberfläche des Zahns.

10. Laserablationsgerät nach einem der Ansprüche 1 bis 9, ferner umfassend:
eine mit der Detektions-Einheit (110) und mit der Laserstrahlquelle (10) verbundene Kontrolleinheit (120) zum Ein- und Ausschalten der Laserstrahlquelle (10) in Abhängigkeit von einem von der Detektions-Einheit (110) gelieferten Signal.

11. Laserablationsgerät nach Anspruch 10, bei welchem die Kontroll-Einheit (120) dafür ausgelegt ist, die Laserstrahlquelle (10) auf einen Ablationsmodus oder einen Diagnosemodus einzustellen, wobei
im Ablationsmodus der gepulste Ablations-Laserstrahl erzeugt wird und im Diagnosemodus ein Diagnose-Laserstrahl erzeugt wird, welcher eine Energiedichte auf der Oberfläche des Zahns aufweist, welche kleiner ist als die Energiedichte, welche zur Ablation des Zahns erforderlich ist.

## Claims

1. Dental laser ablation device for the ablation or removal of tooth material, in particular carious tooth material, comprising:
a laser beam source (1; 10) for providing a pulsed ablation laser beam (50), and
focusing means (2) for focusing the ablation laser beam (50), wherein the ablation laser beam (50) has:
a wavelength of the laser pulses in a range between 700 nm and 1400 nm,
a temporal full width at half maximum of the laser pulses in a range between 5 ps and 100 ps, and
an energy density of the laser pulses at the surface of a tooth in a range between 1.5 J/cm² and 7.5 J/cm²,
**characterized by**
a detection unit (110) for detecting the presence of a signal generated in the tooth or in the surroundings thereof and the strength of said signal, if appropriate,
the detection unit (110) having an optical sensor and **characterized in that** the optical sensor is designed for detecting a second or higher harmonic of an electromagnetic radiation irradiated onto the tooth.

2. Laser ablation device according to Claim 1, in which an energy of the laser pulses is adjusted in a range below 100 µJ, and
a focus of the ablation laser beam (50) at a surface of the tooth is adjusted with a focal diameter in the range between 10 µm and 100 µm.

3. Laser ablation device according to Claim 1 or 2, in which
a repetition rate of laser pulses is adjusted in a range between 500 Hz and 1000 kHz.

4. Laser ablation device according to one of Claims 1 to 3, further comprising:
a laser beam decoupling unit (70), and
a fixing means (150) connected to the laser beam decoupling unit (70) for spatially fixing a distal end of the laser beam decoupling unit (70) with reference to a section of the tooth to be ablated.

5. Laser ablation device according to one of Claims 1 to 4, further comprising:
a beam shaping unit (30) for shaping a substantially rectangular beam profile of the pulsed ablation laser beam (50).

6. Laser ablation device according to one of Claims 1 to 5, further comprising:
a scanning unit (80) for scanning a region of the tooth with the ablation laser beam (50).

7. Laser ablation device according to Claim 6, in which the scanning unit (80) is designed in such a way that exactly one laser pulse is applied to a sub-region covered by the focus of the ablation laser beam (50).

8. Laser ablation device according to Claim 6 or 7, in which the scanning unit (80) is designed in such a way that mutually adjacent sub-regions covered by one laser pulse in each case have a spatial overlap with one another whose surface area is smaller than half a sub-region.

9. Laser ablation device according to one of Claims 1 to 8, further comprising:
an autofocus unit (20) for keeping constant the spatial position of the focus of the ablation laser beam (50) on the surface of the tooth.

10. Laser ablation device according to one of Claims 1 to 9, further comprising:
a control unit (120) connected to the detection unit (110) and the laser beam source (10), for switching the laser beam source (10) on and off in a manner dependent on a signal supplied by the detection unit (110).

11. Laser ablation device according to Claim 10, in which the control unit (120) is designed to set the laser beam source (10) to an ablation mode or a diagnostic mode, the pulsed ablation laser beam being generated in the ablation mode and a diagnostic laser beam being generated in the diagnostic mode that has an energy density on the surface of the tooth which is smaller than the energy density that is required to ablate the tooth.

## Revendications

1. Appareil d'ablation dentaire par laser utilisé pour l'ablation ou l'enlèvement de matière de dent, notamment de matière de dent cariée, comprenant:
une source de rayon laser (1; 10) permettant de mettre à disposition un rayon laser d'ablation (50) pulsé; et
un moyen de focalisation (2) permettant de focaliser le rayon laser d'ablation (50), le rayon laser d'ablation (50) comportant:
une longueur d'onde des impulsions laser située dans une plage comprise entre 700 nm et 1400 nm;
une largeur de période des impulsions laser située dans une plage comprise entre 5 ps et 100 ps; et
une densité volumique d'énergie des impulsions laser sur la surface d'une dent située dans une plage comprise entre 1,5 J/cm² et 7,5 J/cm² ;
**caractérisé par**:
une unité de détection (110) permettant de détecter la présence d'un signal produit dans la dent ou dans ses environs et le cas échéant sa puissance de signal, l'unité de détection (110) comportant un capteur optique, et **caractérisé en ce que** le capteur optique est conçu pour détecter une deuxième harmonique ou une harmonique supérieure d'un rayonnement électromagnétique envoyé sur la dent.

2. Appareil d'ablation par laser selon la revendication 1, dans lequel:
une énergie des impulsions laser est réglée dans une plage inférieure à 100 µJ; et
une focalisation du rayon laser d'ablation (50) sur une surface de la dent est réglée avec un diamètre de focalisation situé dans une plage comprise entre 10 µm et 100 µm.

3. Appareil d'ablation par laser selon la revendication 1 ou 2, dans lequel une vitesse de répétition des impulsions laser est réglée dans une plage comprise entre 500 Hz et 1000 kHz.

4. Appareil d'ablation par laser selon l'une quelconque des revendications 1 à 3, comprenant en outre:
une unité de découplage de rayon laser (70); et
un moyen de fixation (150) relié à l'unité de découplage de rayon laser (70) en vue de fixer dans l'espace une extrémité distale de l'unité de découplage de rayon laser (70) par rapport à une section de la dent à ablater.

5. Appareil d'ablation par laser selon l'une quelconque des revendications 1 à 4, comprenant en outre:
une unité de formation de rayon (30) permettant de former un profil de rayon du rayon laser d'ablation (50) pulsé pour l'essentiel en forme de rectangle.

6. Appareil d'ablation par laser selon l'une quelconque des revendications 1 à 5, comprenant en outre:
une unité de scan (80) permettant de quadriller ou de scanner une région de la dent à l'aide du rayon laser d'ablation (50).

7. Appareil d'ablation par laser selon la revendication 6, dans lequel l'unité de scan (80) est conçue de telle sorte qu'une région partielle détectée par le foyer du rayon laser d'ablation (50) est sollicitée par une impulsion laser précisément.

8. Appareil d'ablation par laser selon la revendication 6 ou 7, dans lequel l'unité de scan (80) est conçue de telle sorte que des zones partielles connexes les unes par rapport aux autres et détectées par respectivement une impulsion laser présentent un chevauchement dans l'espace dont la surface est inférieure à la moitié d'une zone partielle.

9. Appareil d'ablation par laser selon l'une quelconque des revendications 1 à 8, comprenant en outre:
une unité d'autofocalisation (20) permettant de maintenir la position spatiale du foyer du rayon laser d'ablation (50) sur la surface de la dent.

10. Appareil d'ablation par laser selon l'une quelconque des revendications 1 à 9, comprenant en outre:
une unité de contrôle (120) reliée à l'unité de détection (110) et à la source de rayon laser (10) en vue de connecter et déconnecter la source de rayon laser (10) en fonction du signal envoyé par l'unité de détection (110).

11. Appareil d'ablation par laser selon la revendication 10, dans lequel:
l'unité de contrôle (120) est conçue pour régler la source de rayon laser (10) en mode d'ablation ou en mode de diagnostic;
le rayon laser d'ablation pulsé étant produit en mode d'ablation et un rayon laser de diagnostic étant produit en mode de diagnostic, ledit rayon laser de diagnostic présentant une densité volumique d'énergie sur la surface de la dent inférieure à la densité volumique d'énergie nécessaire à l'ablation de la dent.
